# EUROPÄISCHE PATENTANMELDUNG

(11) **EP 0 541 932 A1**
(43) Veröffentlichungstag der Anmeldung: **19.05.1993**
(21) Anmeldenummer: 92115899.4
(22) Anmeldetag: 17.09.1992
(51) Int. Cl.: A61B 1/00

(54) **Vorrichtung zum Befestigen und Halten eines Hilfsinstrumentes an einem medizinischen Instrument**

(30) Priorität: 14.11.1991 DE 4137426
(71) Anmelder: Richard Wolf GmbH, D-75438 Knittlingen (DE)
(72) Erfinder: Heckele, Helmut, W-7134 Knittlingen (DE)
(74) Vertreter: Vollmann, Heiko, Dipl.-Ing.

(57) **Zusammenfassung**

Die Vorrichtung dient zum Befestigen und Halten eines Hilfsinstrumentes an einem medizinischen Instrument, durch dessen Schaft (4) ein Hilfsinstrument (13) eingeführt werden kann. Die Vorrichtung hat eine proximalseitig am Schaft (4) lösbar festlegbare Aufnahme (1) für ein Kupplungsteil (14) des Hilfsinstrumentes und ein Spannelement (3), das relativ zur Längsachse der Aufnahme verstellt werden kann und bei Verstellung kraftschlüssig gegen den Außenumfang der zwischen Aufnahme (1) und Spannelement (3) befindlichen Wand des Schaftes (4) zur Anlage gebracht wird.

## Beschreibung

Die Erfindung bezieht sich auf eine Vorrichtung zum Befestigen und Halten eines Hilfsinstrumentes an einem medizinischen Instrument, durch dessen Schaft ein Hilfsinstrument eingeführt werden kann.

Bei der Anwendung einfacher Endoskope, die im wesentlichen nur aus einem hohlen bzw. mit einem Durchführungskanal versehenen Schaft bestehen, ist es oft erforderlich, Hilfsinstrumente einzuführen und am proximalen Schaftende zu befestigen und zu halten. Solche Hilfsinstrumente können z. B. Lichtleitstäbe, Optiken oder Operationsinstrumente sein.

Es ist z. B. bei Bronchoskopen und Laryngoskopen durch die GB 1 150 016 A bekannt, am proximalen Schaftende einen radial nach außen verlaufenden Vierkantstab anzubringen, auf den von außen her eine mit einer entsprechenden Vierkanthülse versehene Beleuchtungseinrichtung aufgeschoben und festgeklemmt wird, welche dann durch eine Umfangsausnehmung des Schaftes in dessen Inneres hineinragt. Der eigentliche Lichtleitstab wird bei dieser Ausführung mit einem Steckstift in eine achsparallel zum Schaft verlaufende Bohrung des Vierkantstabes eingesteckt.

Weiterhin ist es bekannt, das proximale Schaftende in Achsrichtung einzuschlitzen und in diesen Schlitz einen abgewinkelten Lichtleitstab einzuhängen, welcher zusätzlich wiederum mit einem Steckstift in einer Adapterbohrung am Schaftende mittels eines Kugelrastelementes gesichert wird. Auch das Sichern eines solchen Steckstiftes durch Federbacken oder Stellschrauben ist bekannt.

Zur Befestigung einer Beleuchtungseinrichtung am proximalen Schaftende ist bei einem anderen bekannten Instrument eine radial oder winkelig zum Schaft verlaufende und mit dem Schaftinneren in Verbindung stehende Buchse vorgesehen, in die die Beleuchtungseinrichtung von außen her einsteckbar ist und mit einem kleinen Prisma oder Spiegel in das Schaftinnere ragt.

Da diese Befestigungseinrichtungen immer nur zum Halten eines bestimmten Hilfsinstrumentes in einer unveränderlichen Lage am Schaft geeignet sind, verwendet man bei Bronchoskopen schon mehrere einzeln oder hintereinander auf den Schaft aufsteckbare Adapterhülsen. Wenn auch das Hilfsinstrument bei dieser Ausführung in radialer Richtung festgelegt werden kann, so besteht doch ein wesentlicher Nachteil darin, daß bei der Handhabung des Instrumentes ein unbeabsichtigtes Lösen der aufgesteckten Adapterhülsen und der Instrumenteile möglich ist.

Es ist Aufgabe der Erfindung, eine Vorrichtung zur Befestigung von in den Schaft einzuführenden Hilfsinstrumenten zu schaffen, die nicht auf eine bestimmte Art und Ausführung dieser Instrumente festgelegt ist, also u. a. auch bei über den Umfang geschlossenen oder geteilten Instrumentenschäften anwendbar ist, und die an Instrumentenschäften unterschiedlicher Querschnitte in beliebiger Stellung anbringbar ist. Weiterhin soll die Vorrichtung die Handhabung des Instrumentes nicht behindern.

Entsprechend dieser Aufgabe sieht die Erfindung, ausgehend von der eingangs erwähnten Vorrichtung, eine proximalseitig am Schaft lösbar festlegbare Aufnahme für ein Kupplungsteil des Hilfsinstrumentes und ein Spannelement vor, das radial in bezug auf die Längsachse der Aufnahme verstellbar ist und bei Verstellung in Richtung auf die Aufnahme kraftschlüssig gegen den Außenumfang des zwischen Aufnahme und Spannelement befindlichen Schaftwand zur Anlage bringbar ist.

Diese den proximalen Schaftrand überfassende Vorrichtung ist auf Instrumentenschäften mit unterschiedlichen Querschnitten, z. B. auch an einem Spreizlaryngoskop, befestigbar. Da sie nur den Schaftrand zum Festklemmen benötigt, kann sie in beliebiger radialer Winkelstellung angebracht werden. Darüberhinaus ist die Anbringung mehrerer Befestigungsvorrichtungen auf dem Schaftrand möglich.

Ein weiterer Vorteil der Erfindung ist, daß die im Schaftinneren liegende Aufnahme Hilfsinstrumente beliebiger Bauart aufnehmen kann, z. B. Lichtleitstäbe direkt durch die Aufnahme hindurchgeführt werden können, ebenso wie andere Hilfsinstrumente mit geringem Durchmesser, während sonstige Hilfsinstrumente mit Hilfe eines Kupplungsteiles befestigbar sind.

Das Spannelement kann als ein von Hand verstellbarer Keil ausgebildet werden, der eine parallel zur Längsachse der Aufnahme verlaufende Spannfläche und eine hierzu schräg verlaufende Gleitfläche hat, mit der er sich auf einer entsprechend schrägen Gleitfläche im Vorrichtungsgehäuse abstützt. Weiterhin hat der Keil eine vertikal zur Längsachse der Aufnahme verlaufende Angriffsfläche, gegen die das Ende einer exzentrisch zur Aufnahme angeordneten Stellschraube drückt.

Um den Schaft des Instrumentes sicher zwischen Keil und Aufnahme einspannen zu können, sollte die Spannfläche des Spannelementes einen konvexen und die dem Spannelement gegenüberliegende Fläche der Aufnahme einen konkaven Verlauf haben, wobei die Radien dieser Flächen kleiner sind als der Radius des Instrumentenschaftes. Um die Vorrichtung sicher am Schaftrand festlegen zu können, kann die Spannfläche des Spannelementes oberflächenbeschichtet oder angerauht sein, um dadurch die Reibung zu erhöhen.

Wenn das in die Aufnahme eingeführte Hilfsinstrument gegen Verdrehen und gegen axiale Verschiebung gesichert werden soll, hat der Durchgang der Aufnahme eine von einer Kreisfläche abweichende Querschnittsfläche, in die ein am Hilfsinstrument vorgesehenes Kupplungsteil formschlüssig und verdrehsicher eingreift, während außerdem in den Durchgang ein federnd nach außen auslenkbarer Vorsprung ragt, der auf den Umfang des Kupplungsteiles drückt und das Hilfsinstrument kraftschlüssig in der Aufnahme festlegt.

Anhand der Zeichnungen wird ein Ausführungsbeispiel der Erfindung nachstehend näher beschrieben. Es zeigt:
- Figur 1: eine Seitenansicht der auf einen Schaftrand aufzusetzenden Vorrichtung,
- Figur 2: eine Aufsicht auf die Vorrichtung nach Figur 1,
- Figur 3: eine Stirnansicht der Vorrichtung vom distalen Ende her,
- Figur 4: eine Seitenansicht eines Laryngoskop mit eingesetzter Vorrichtung und einer von dieser gehaltenen Optik nach Figur 5,
- Figur 5: eine Ansicht einer Endoskop-Optik mit seitlichem Kupplungsteil und
- Figur 6: eine Ansicht eines Lichtleitstabes.

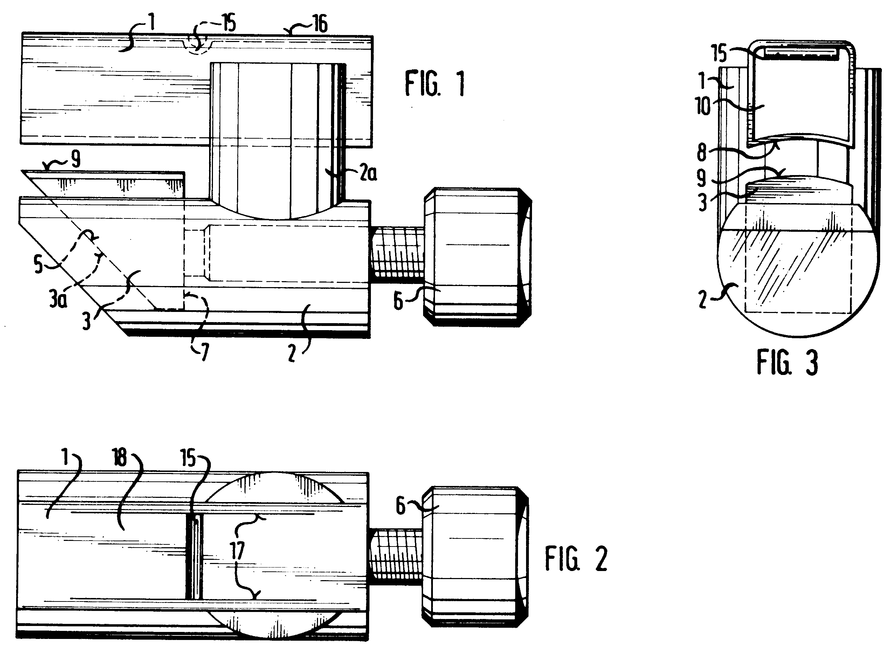

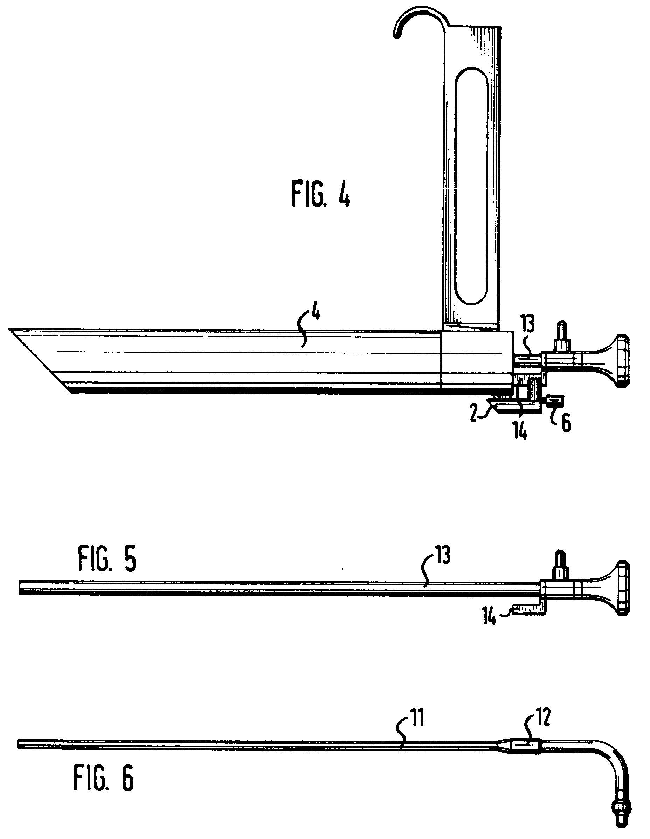

Die in den Figuren 1 bis 3 dargestellte Vorrichtung besteht aus einer Aufnahme 1 und einem Grundkörper 2 mit Spannelement 3 als Teile des Vorrichtungsgehäuses. Die Aufnahme 1, die in das freie Lumen des Schaftes 4 eines Instrumentes gemäß Figur 4 eingreift, ist eine Hülse, die noch später weiter beschrieben wird. Sie ist fest mit dem Grundkörper 2 über einen Steg 2a verbunden.

Der Grundkörper 2 trägt an seinem distalen Teil das Spannelement 3. Dieses ist im Ausführungsbeispiel ein Keil, der in einer entsprechend geformten Ausnehmung des Grundkörpers sitzt und gemäß Figur 1 mit einer schräg gestellten Gleitfläche 3a gegen eine entsprechend schräge Gleitfläche 5 des Grundkörpers anliegt. Eine Stellschraube 6 ist in einem Gewinde des Grundkörpers von Hand verdrehbar gelagert. Dreht man die Schraube in das Gewinde des Grundkörpers hinein, so wird der Keil 3 auf der Gleitfläche 5 relativ zur Aufnahme 1 hin verschoben, wodurch schließlich der Schaft 4 des Instrumentes zwischen dem Keil 3 und dem gegenüberliegenden Außenumfang der Aufnahme 1 festgelegt wird. Dabei greift das freie Ende der Stellschraube 6 an der vertikal zur Längsachse der Aufnahme 1 verlaufenden Druckfläche 7 des Keiles 3 an.

Vom Ausführungsbeispiel abweichend kann das Spannelement anstelle des Keiles 3 auch eine direkt wirkende Spannschraube oder ein Exenter sein. Weiterhin könnte anstelle des Keiles und der Stellschraube auch ein Kniehebel zur Anwendung kommen.

Die dargestellte Stellschraube 6, der Grundkörper 2 und das Spannelement 3 befinden sich exzentrisch zur Aufnahme 1 und somit ausserhalb des Instrumentenschaftes 4, so daß sie beim Einsetzen von Hilfsinstrumenten und bei der sonstigen Handhabung des medizinischen Instrumentes nicht im Wege stehen.

Zwischen der Aufnahme 1 und dem Spannelement 3 wird die Wandung des Schaftes 4 gemäß Figur 4 eingespannt, so daß die Vorrichtung den proximalen Schaftrand übergreift. Sie kann dort an jeder beliebigen Position angebracht werden, so daß bei Bedarf ggf. auch mehrere dieser Vorrichtungen am proximalen Ende des Instrumentenschaftes befestigt werden können.

Der Schaft 4 kann sowohl kreisförmigen als auch ovalen, abgeplatteten oder sonstigen Querschnitt haben. Damit die Vorrichtung auf jeden Fall einen festen Sitz bekommt, ist für die Aufnahme 1 an der dem Spannelement 3 zugekehrten Seite 8 ein konkaver Verlauf vorgesehen, während das Spannelement eine konvexe Spannfläche 9 aufweist. Dabei sind die Radien dieser Flächen 8, 9 kleiner als der Radius des Schaftes 4, um sicherzustellen, daß sich drei definierte Einspannlinien zwischen den Teilen 1, 3 und 4 ergeben, die in Achsrichtung des Schaftes 4 verlaufen und daher die Vorrichtung sicher zentrieren. Diese Wirkung wird auch erreicht, wenn die Spannfläche des Keiles konkav und die gegenüberliegende Fläche der Aufnahme konvex verläuft.

Der Durchgang der Aufnahme 1 kann einen kreisförmigen Querschnitt haben, so daß sich Hilfsinstrumente mit rundem Schaft gut in und durch die Aufnahme schieben lassen. In diesem Fall würde ein Bereich des Hilfsinstrumentenschaftes das "Kupplungsteil" bilden. Wenn es allerdings auf eine Sicherung der Hilfsinstrumente gegen Verdrehung ankommt, ist es zweckmäßig, einen vom runden Querschnitt abweichenden Durchgang 10, also beispielsweise einen im wesentlichen quadratischen Querschnitt, zu wählen, wie es in Figur 3 dargestellt ist. In diesen Durchgang 10 lassen sich zwar auch Hilfsinstrumente mit runden Querschnitten einschieben, es ist dann aber entsprechend Figur 6 außerdem möglich, einen Lichtleitstab 11 oder ein anderes Hilfsinstrument am proximalen Ende mit einem korrespondierenden Vierkant 12 als Kupplungsteil zu versehen, der nach Einführung des Hilfsinstrumentes in die Aufnahme formschlüssig und verdrehsicher im Durchgang 10 sitzt.

In der Figur 5 ist demgegenüber und stellvertretend für sonstige Hilfsinstrumente eine Optik 13 dargestellt, die ein seitliches Kupplungsteil 14 aufweist, dessen Querschnitt dem des Durchganges 10 angepaßt ist und der nach Einführen des Hilfsinstrumentes in den Schaft 4 formschlüssig in der Aufnahme 1 steckt, während die Optik als solche außerhalb der Aufnahme durch den Instrumentenkanal verläuft.

Neben der vorstehend beschriebenen Sicherung gegen Verdrehen des Hilfsinstrumentes kann auch eine Sicherung gegen axiales Herausgleiten des Kupplungsteiles aus der Aufnahme notwendig werden. Hierzu wird zweckmäßigerweise so vorgegangen, daß diese Sicherung mit Hilfe eines oder mehrerer Federelemente erfolgt, und zwar beispielsweise durch einen in den Durchgang 10 der Aufnahme 1 ragenden Vorsprung 15, der beim Einstecken des Kupplungsteiles 12 oder 14 in die Aufnahme radial und federnd nach außen ausgelenkt wird und gegen den Umfang des Kupplungsteiles drückt, um dieses kraftschlüssig mit der Aufnahme zu verbinden. Es besteht auch die Möglichkeit, daß der Vorsprung 15 in eine im Kupplungsteil 14 befindliche Nut eingreift, wodurch ein axiales Herausgleiten des Kupplungsteiles 14 aus der Aufnahme 1 wirksam verhindert wird.

Die erwähnte Federwirkung kann dadurch erreicht werden, daß die obere Wandung 16 der Aufnahme 1 mit zwei Längsschlitzen 17 versehen wird, so daß der zwischen den Schlitzen befindliche Teil eine Blattfeder 18 bildet. Der Vorsprung 15 kann durch eine Sicke in dieser Blattfeder gebildet sein, die im übrigen auch mehrteilig ausgebildet sein und mehr als eine Sicke aufweisen kann.

## Patentansprüche

1. Vorrichtung zum Befestigen und Halten eines Hilfsinstrumentes an einem medizinischen Instrument, durch dessen Schaft ein Hilfsinstrument geführt werden kann, gekennzeichnet durch eine proximalseitig am Schaft lösbar festlegbare Aufnahme (1) für ein Kupplungsteil (12, 14) des Hilfsinstrumentes (11, 13) und durch ein Spannelement (3), das relativ zur Längsachse der Aufnahme verstellbar ist und bei Verstellung kraftschlüssig gegen den Außenumfang der zwischen Aufnahme und Spannelement befindlichen Wand des Schaftes (4) zur Anlage bringbar ist.

2. Vorrichtung nach Anspruch 1, dadurch gekennzeichnet, daß das Spannelement ein verstellbarer Keil (3) ist.

3. Vorrichtung nach Anspruch 2, dadurch gekennzeichnet, daß der Keil (3) eine parallel zur Längsachse der Aufnahme (1) verlaufende Spannfläche (9) und eine hierzu schräg verlaufende Gleitfläche (3a) hat, mit der er sich auf einer entsprechend schrägen Gleitfläche (5) im Vorrichtungsgehäuse abstützt, und daß der Keil eine vertikal zur Längsachse der Aufnahme verlaufende Angriffsfläche (7) hat, an der das freie Ende einer exzentrisch zur Aufnahme angeordneten Stellschraube (6) angreift.

4. Vorrichtung nach einem der vorhergehenden Ansprüche, dadurch gekennzeichnet, daß die Spannfläche (9) des Spannelementes (3) einen konvexen und die dem Spannelement gegenüberliegende Fläche (8) der Aufnahme einen konkaven Verlauf hat und daß die Radien dieser Flächen (8, 9) kleiner sind als der Radius des Instrumentschaftes (4).

5. Vorrichtung nach einem der vorhergehenden Ansprüche, dadurch gekennzeichnet, daß der Durchgang (10) in der Aufnahme (1) einen beliebigen Querschnitt aufweist.

6. Vorrichtung nach einem der vorhergehenden Ansprüche, dadurch gekennzeichnet, daß in den Durchgang (10) der Aufnahme (1) zumindest ein vom Kupplungsteil (12, 14) des Hilfsinstrumentes (11, 13) gegen Federwirkung nach außen auslenkbarer Vorsprung (15) ragt.

7. Vorrichtung nach einem der vorhergehenden Ansprüche, dadurch gekennzeichnet, daß der Vorsprung aus einer Sicke (15) an einer Blattfeder (18) besteht, die durch zwei parallele Schlitze (17) in der Wandung (16) der Aufnahme (1) gebildet ist.
